(19) Europäisches Patentamt
European Patent Office
Office européen des brevets

(11) Publication number: **0 068 324 A2**

(12) # EUROPEAN PATENT APPLICATION

(21) Application number: **82105305.5**

(22) Date of filing: **16.06.82**

(51) Int. Cl.³: **A 61 K 9/50**, A 61 K 31/495

(30) Priority: **19.06.81 JP 95536/81**

(43) Date of publication of application: **05.01.83 Bulletin 83/1**

(84) Designated Contracting States: **CH DE FR GB LI NL**

(71) Applicant: **Tanabe Seiyaku Co., Ltd., No. 21 Dosho-machi 3-chome Higashi-ku, Osaka-shi Osaka-fu (JP)**

(72) Inventor: **Samejima, Masayoshi, No. 2515-23, Oaza-Aomadani, Minoh-shi Osaka-fu (JP)**
Inventor: **Hirata, Goichi, No. 13-9, Nishiyama Adachi, Yawata-shi Kyoto-fu (JP)**
Inventor: **Koida, Yoshiyuki, No. 36-6, Amanogahara-cho 2-chome, Katano-shi Osaka-fu (JP)**

(74) Representative: **Kraus, Walter, Dr. et al, Patentanwälte Dres. Kraus & Weisert Irmgardstrasse 15, D-8000 München 71 (DE)**

(54) **Microcapsule preparation for reducing increased intracranial pressure and a process for preparing the same.**

(57) A microcapsule preparation for reducing increased intracranial pressure, which contains as an effective ingredient a propanol derivative of the formula:

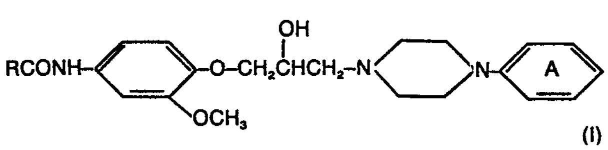

(I)

wherein R is an alkyl having 1 to 4 carbon atoms, and ring A is phenyl, halogenophenyl, methylphenyl or trifluoromethylphenyl, or a pharmaceutically acceptable acid addition salt thereof which is coated with ethyl cellulose film, and a process for preparing the microcapsule preparation by forming a coating film of ethyl cellulose onto the core material of the propanol derivative (I) or a salt thereof utilizing phase separation phenomenon which arises in cooling of a dispersion of the core material in a hot organic solvent solution of ethyl cellulose.

EP 0 068 324 A2

# MICROCAPSULE PREPARATION FOR REDUCING INCREASED INTRACRANIAL PRESSURE AND A PROCESS FOR PREPARING THE SAME

The present invention relates to a microcapsule preparation suitable for reducing increased intracranial pressure and a process for preparing the microcapsule preparation. More particularly, it relates to a microcapsule preparation containing as an effective ingredient a propanol derivative of the formula:

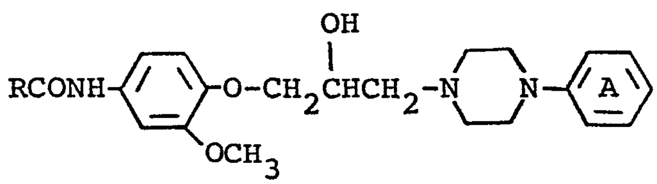

(I)

wherein R is an alkyl having 1 to 4 carbon atoms, and ring A is phenyl, halogenophenyl, methylphenyl or trifluoromethylphenyl, or a pharmaceutically acceptable acid addition salt thereof, and a process for preparing the microcapsule preparation.

It is known that an increase of intracranial pressure is induced by an increase in volume of various intracranial components such as blood, spinal fluid and cerebral parenchymatous fluid, i.e. increase of hematoma or tumor, increase of spinal fluid due to disorders of absorption and secretion, or increase in volume of cerebral parenchymatous fluid due to acute tumor or edema. For instance, various cerebral diseases,

such as cerebral hemorrhage, subarachnoid hemorrhage, cerebral thrombosis, cerebral embolism, head injury, cerebral tumor and encephalomyelitis, induce the increase in intracranial pressure. Moreover, it is known that the increased intracranial pressure affects adversely not only on the injured tissues but also on normal cerebral tissues, which results in disturbances of cerebral circulation, and hence, it exerts serious adverse effects on the recovery or prognosis of patients or is sometimes fatal to them. Accordingly, it is very important to give the most suitable and effective treatment to the patients suffering from increased intracranial pressure not only at the first stage of the diseases but also during the treatment and prognosis thereafter.

An instillation of a hypertonic solution of glycerol or mannitol has hitherto been used for the treatment of patients suffering from increased intracranial pressure. The injection of the hypertonic solution raises blood osmolality and creates an osmotic gradient between blood plasma and cerebral parenchymatous fluid or spinal fluid with resultant net removal of water from brain. Thus, the therapeutic effect of injection of hypertonic solution on the increased intracranial pressure is based on the hyperosmolar dehydrating effect, and hence, in order to obtain the desired therapeutic effect, a large amount (500 to 1,000 ml/day) of a high concentration of the solution (osmotic ratio: 4 to 5 times) must be

intravenously administered to the patients. Such an intravenous injection of the large amount of hypertonic solution causes disadvantageously haematological abnormalities (e.g. disturbance of water-electrolyte equilibrium, haemolysis, decrease in haematocrit level), and further, owing to leak of the hypertonic solution from blood tube at the injured area in brain, it results in a rebound increase in the intracranial pressure to levels higher than those that existed before therapy, which causes bad prognosis of the patients.

As a result of intensive study on a medicine useful for the treatment of increased intracranial pressure, the present inventors had found that the propanol derivative (I) shows excellent activity of reducing increased intracranial pressure (cf. U.S. Patent 4,307,097). Further study has been done on the preparations of the compound. As a result, it has been found that a microcapsule preparation thereof is particularly useful and can effectively be administered to the patients in oral route without pain like in injection route and it is easy to control the amount of release of the active ingredient from the preparation so as to exhibit the most suitable effect in accordance with the kind and symptom of individual patient, and further that the desired microcapsule preparation can easily be prepared by forming a coating film of ethyl cellulose on particles of the active ingredient by

utilizing phase separation phenomenon of a solution of ethyl cellulose in an organic solvent.

An object of the present invention is to provide a microcapsule preparation useful for the treatment of increased intracranial pressure. Another object of the invention is to provide a microcapsule preparation of the propanol derivative (I) suitable for the therapeutic treatment of increased intracranial pressure and other various diseases induced thereby without undesirable side effects such as haematological abnormalities, rebound increase in the intracranial pressure, etc. which are observed in the intravenous injection of a hypertonic solution of glycerol or mannitol. A further object of the invention is to provide a preparation of the propanol derivative (I) which can easily be administered to the patients suffering from increased intracranial pressure in oral route. Still further object of the invention is to provide a process for preparing the microcapsule preparation. These and other objects and advantages of the invention will be apparent to persons skilled in the art from the following description.

The microcapsule preparation for reducing increased intracranial pressure of the present invention comprises microparticles of a propanol derivative (I) or a pharmaceutically acceptable acid addition salt thereof which are coated by ethyl cellulose film and

can be prepared by dispersing uniformly microparticles of the propanol derivative (I) or a pharmaceutically acceptable acid addition salt thereof into a hot solution of ethyl cellulose (a film-forming material) in an organic solvent and cooling the dispersion, whereby ethyl cellulose is deposited onto the microparticles owing to phase separation to form coating film thereon.

The propanol derivative (I) of the present invention includes a compound of the formula (I) wherein R is an alkyl having 1 to 4 carbon atoms (e.g. methyl, ethyl, propyl, butyl), and the ring A is phenyl, methylphenyl (e.g. 2-methylphenyl), chlorophenyl (e.g. 2-chlorophenyl, 3-chlorophenyl, 4-chlorophenyl), fluorophenyl (e.g. 2-fluorophenyl, 3-fluorophenyl, 4-fluorophenyl), or trifluoromethylphenyl (e.g. 3-trifluoromethylphenyl). Preferred compounds are a compound of the formula (I) wherein R is methyl, and the ring A is phenyl, chlorophenyl, or fluorophenyl. Particularly preffered compounds are a compound of the formula (I) wherein R is methyl, and the ring A is 2-chlorophenyl, 2-fluorophenyl, or 3-fluorophenyl. Specifically suitable compound is 1-(4-acetamido-2-methoxyphenoxy)-3-[4-(3-fluorophenyl)-piperazino]-2-propanol.

The propanol derivative (I) may be used in the form of a free base or in the form of a pharmaceutically acceptable acid addition salt. The acid addition salt include a salt of an inorganic acid (e.g. hydrochloric

acid, phosphoric acid, nitric acid, sulfuric acid), and an organic acid (e.g. acetic acid, lactic acid, citric acid, fumaric acid, maleic acid, glycine, aspartic acid, methanesulfonic acid, benzoic acid).

The microcapsule preparation for reducing increased intracranial pressure of the present invention can easily be prepared by a conventional microencapsulation method using ethyl cellulose. That is, a film-forming material, ethyl cellulose is dissolved with heating in an appropriate organic solvent, and thereto is uniformly dispersed microparticles of an effective propanol derivative (I) or a salt thereof, and then the dispersion is cooled, whereby a coating film of ethyl cellulose is formed onto the microparticles of the effective propanol derivative (I) or a salt thereof owing to phase separation such as coacervation or flocculation.

The organic solvent used in the present invention is a poor solvent to the propanol derivative (I) or a salt thereof and is a good solvent in hot state but a poor solvent in cooled state to ethyl cellulose. Suitable examples of the organic solvent are cyclohexane or a mixture of cyclohexane and n-hexane, among which cyclohexane is particularly preferable.

In the present invention, the propanol derivative (I) or a salt thereof is used as a core for the

microcapsule. The particle size of the core material is not critical, but is usually in the range of about 30 to 1,000 $\mu$, preferably 50 to 500 $\mu$. The propanol derivative (I) or a salt thereof may be used after being granulated with conventional diluents (e.g. lactose, starch, mannitol, microcrystalline cellulose, etc.) and binders (e.g. polyvinyl alcohol, polyvinyl acetate, hydroxypropyl cellulose, dextrine, gum arabic, etc), wherein the active propanol derivative (I) or a salt thereof is contained in an amount of 3 to 40 % by weight.

The ethyl cellulose for forming microcapsule film on the core material has preferably an ethoxy content of 46.5 to 55 % and a viscosity of 3 to 500 cP (at 25°C in a 5 % solution of ethyl cellulose in a mixed solvent of toluene : ethanol = 4 : 1). The ethyl cellulose is used in an amount of 0.05 to 5 times by weight of the amount of the core material.

The ethyl cellulose as mentioned above is dissolved with heating in an organic solvent, and to the hot organic solvent solution is uniformly dispersed with stirring the core material at a temperature of about 55 to 75°C. The resulting dispersion of core material is cooled, whereby a phase separation arises. The phase separation is carried out in the presence or absence of a phase-separation-inducing agent, which is done by the phenomenon of so-called coacervation or

flocculation. In this phase separation, a wall film-forming auxiliary and a surface active agent may also optionally be used. The phase-separation-inducing agent includes, for example, polyethylene, butyl rubber, polybutadiene, and polyisobutylene. The wall film-forming auxiliary includes, for example, an organic silicon polymer such as dimethylpolysiloxane, methylphenylpolysiloxane, diphenylpolysiloxane, and polystyrene-polydimethylsiloxane block copolymer which may be used alone or in a mixture of two or more thereof, or in a mixture of these organic silicon polymer and 1 to 50 % by weight of an additive such as silicon dioxide, titanium oxide or calcium stearate. The surface active agent includes, for example, sorbitan fatty acid ester, glycerine fatty acid ester, propylene glycol fatty acid ester, soy bean phosphatide, yolk phosphatide, and calcium stearyllactate. These additives may be added to the solution of ethyl cellulose when ethyl cellulose is dissolved in an organic solvent. The amount of these additives is preferably in the range of 0.01 to 10 % by weight as to the phase-separation-inducing agent, 0.01 to 10 % by weight as to the wall film-forming auxiliary, and about 0.003 to 10 % by weight as to the surface active agent, based on the weight of the solution of ethyl cellulose. The phase separation of ethyl cellulose may be done, for example, by cooling the solution from about 80°C to

room temperature at a rate of 0.05 to 4°C per minute, by which ethyl cellulose wall film is formed around the core materials to obtain stably solidified coated particles.

The microcapsule thus obtained can be separated conventional separation methods, for example, decantation, filtration or centrifuge, without mutual adhesion or aggregation of the microcapsules. The microcapsules are optionally washed with cyclohexane, petroleum ether, or n-hexane and then dried by conventional drying methods, such as hot-air drying or heat transfer drying.

According to the present invention, the thickness of the coating film may optionally be varied by changing the proportion of the core material and ethyl cellulose, and it has advantages such as sustained and delayed release of the active propanol derivative (I) or a salt thereof, protection of the active ingredient and prevention of alteration thereof due to contact with other ingredients by the non-hygroscopic coating film of ethyl cellulose, and further masking or improvement of the peculiar unpleasant taste of the propanol derivative (I).

The following experiments are illustrative of release of active ingredient from the microcapsule and taste or feeling thereof when administered.

Experiment

Microcapsule preparations containing 1-(4-acetamido-2-methoxyphenoxy)-3-[4-(3-fluorophenyl)-piperazino]-2-propanol hydrochloride were prepared in the following manner by varying the mixed ratio of the core material and ethyl cellulose, and there were measured yield of microcapsule, content of the propanol derivative in the microcapsules, bitter taste of the preparation when administered, and change of releasing rate of the propanol derivative with laspe of time during keeping it in water at 37°C.

Method:

1) Core material

To a powdery mixture of 1-(4-acetamido-2-methoxyphenoxy)-3-[4-(3-fluorophenyl)piperazino]-2-propanol hydrochloride (27 parts by weight) and lactose (70 parts by weight) was added a solution of methyl cellulose (2 parts by weight) in water (20 parts by weight). The mixture was kneaded and granulated by conventional method and then dried, followed by regulating the particles size to obtain particles of core material having a particle size of 105 to 350 $\mu$.

2) Preparation of microcapsule

In cyclohexane (600 ml) were dissolved a silicon resin (as defined in 4th Food Additives Formulary, viscosity: 100 - 1,100 cSt, a mixture of dimethyl polysiloxane and 3 to 15 % by weight of silicon

dioxide) (18 g) and soy bean phosphatide (0.6 g). To the solution was added ethyl cellulose (ethoxy content: 48.5 %, viscosity: 100 cP) in an amount as shown in Table 1 and it was dissolved by heating at 80°C. To the solution was added the core material (36 g), and the mixture was cooled to room temperature with stirring at 300 rpm. The resulting microcapsules were separated, washed and dried. The microcapsules thus obtained were passed through the JIS standard sieve (aperture: 500 μ), and the microcapsules remained on the JIS standard sieve (aperture: 105 μ) were collected to give microcapsules containing propanol derivative which conformed to the fine granule standard as defined in the 10th Edition of the Pharmacopoeia of Japan (hereinafter, referred to merely as "fine granule standard").

3) Results

Various properties of the microcapsules obtained above are shown in Table 1 and attached Figure 1.

Table 1

| Expt. No. | Amount of ethyl cellulose (g) | Capsule conformed to the fine granule standard | | Time of release in 50 % (minute) | Bitter taste when admin-istered |
|---|---|---|---|---|---|
| | | Yield (g) | Content of propanol derivative (%) | | |
| A | 3.6 | 38.4 | 24.4 | 12 | Non |
| B | 6 | 41.0 | 23.0 | 54 | Non |
| C | 12 | 47.0 | 20.7 | 138 | Non |
| Control | 0 | - | 26.8 | 3 | Bitter |

Figure 1 shows the change of releasing rate of the active ingredient (when kept in water at 37°C) of the microcapsule preparation of the present invention and a reference preparation having no coating film of ethyl cellulose.

The microcapsule preparation for reducing increased intracranial pressure of the present invention contains 3 to 98 % by weight of the active propanol derivative (I) or a pharmaceutically acceptable salt thereof, and the dose of the preparation may vary with age, body weight of patients and severity of diseases, but is usually in the range of 30 to 150 mg per day, and the preparation is orally administered from one to three times per day.

The microcapsule preparation containing a propanol derivative of the present invention can reduce

the intracranial pressure in an extremely small amount, which may be by a different mechanism from the hyperosmolar dehydrating effect by the known intravenous injection of a large amount of hypertonic solution, and the therapeutic effect is large and durable without showing any side effect such as a rebound increase of the intracranial pressure. The propanol derivative (I) of the present invention is low toxic and highly safeful. For example, 1-(4-acetamido-2-methoxyphenoxy)-3-[4-(3-fluorophenyl)piperazino]-2-propanol shows $LD_{50}$ of about 1,500 mg/kg in mice when measured one week after it was orally administered.

As mentioned hereinbefore, the propanol derivative (I) of the present invention shows a high activity of reducing intracranial pressure with high safety and hence is useful for the treatment of patients suffering from increased intracranial pressure accompanied with various cerebral diseases such as cerebral infarct (e.g. cerebral thrombosis, cerebral embolism), cerebral hemorrhage, subarachnoid hemorrhage, head injury, cerebral tumor, encephalomyelitis, or cerebral edema. Although the increase in intracranial pressure promotes topical or whole cerebral edema to induce disturbances of cerebral circulation accompanying variation of blood pressure, which results in more increase of intractranial pressure, the microcapsule containing the propanol derivative (I) of the present

invention can interrupt such a vicious circle of cerebral diseases because of its potent intracranial pressure-lowering effect and can prohibit the progress of cerebral edema and can improve also the disturbances of cerebral circulation. Accordingly, the microcapsule preparation of the present invention can also be used for the treatment of various intracranial diseases as mentioned above. Moreover, the microcapsule preparation containing a propanol derivative (I) of the present invention can also be used for the treatment of consciousness disorder, nervous disturbance, or subjective symptoms (e.g. dizziness, headache, nausea, vomiting) and hence is useful for prophylaxis and treatment of increased intracranial pressure during or after brain surgical operation. Since the propanol derivative (I) has a significant activity of increasing blood flow in brain in addition to the reducing intracranial pressure, the microcapsule preparation of the present invention can also be used for the improvement of cerebral circulation, particularly at ischemic portion in brain.

The propanol derivative (I) of the present invention can be prepared by reacting 3-(4-acylamido-2-methoxyphenoxy)-1,2-epoxypropane (II) with a piperazine derivative (III) in the same manner as is described in Japanese Patent Publication (unexamined) No. 21127/1978 as shown in the following reaction scheme:

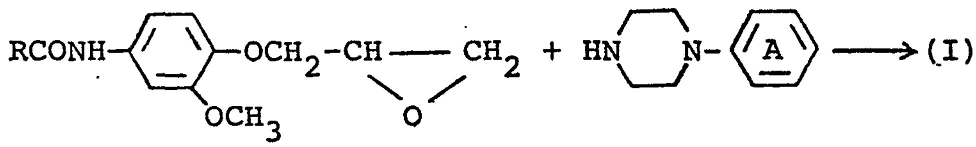

(II) (III)

wherein R and the ring A are as defined above.

The preparation of the propanol derivative (I) and microcapsule preparations of the present invention will be illustrated by the following Preparation and Examples.

Preparation 1

To a solution of 3-(4-acetamido-2-methoxyphenoxy)-1,2-epoxypropane (1.0 g) in ethanol (40 ml) was added 4-phenylpiperazine (750 mg), and the mixture was stirred at toom temperature for 18 hours. The reaction mixture was concentrated under reduced pressure to remove ethanol. The resulting residue was recrystallized from a mixture of ethyl acetate-methanol to obtain 1-(4-acetamido-2-methoxyphenoxy)-3-(4-phenylpiperazino)-2-propanol (1.55 g) as colorless needles, m.p. 154.5 - 156°C.

The hydrochloride of this product: m.p. 242 - 244°C (dec.)

The methanesulfonate of this product: m.p. 191 - 193°C

Preparation 2

To a solution of 3-(4-acetamido-2-methoxyphenoxy)-1,2-epoxypropane (800 mg) in ethanol (40 ml)

was added 4-(2-fluorophenyl)piperazine (670 mg), and the mixture was refluxed for 4 hours. The reaction mixture was concentrated under reduced pressure to remove ethanol. The resulting residue was recrystallized from a mixture of isopropanol-isopropyl ether to obtain 1-(4-acetamido-2-methoxyphenoxy)-3-[4-(2-fluorophenyl)piperazino]-2-propanol (770 mg) as colorless crystalline particles, m.p. 143.5 - 144.5°C.

The mother liquor produced in the above procedure was concentrated to driness, and the residue was purified by silica gel chromatography (solvent: 2 % methanol/chloroform), and the resulting crystals were recrystallized in the same manner as described above, by which the same product as above (460 mg) was further obtained.

Preparation 3 to 9

In the same manner as described in the above Preparations 1 and 2, the following compounds were prepared.

(3) 1-(4-Acetamido-2-methoxyphenoxy)-3-[4-(2-methylphenyl)piperazino]-2-propanol, m.p. 126.5 - 128°C.

(4) 1-(4-Acetamido-2-methoxyphenoxy)-3-[4-(4-chlorophenyl)piperazino]-2-propanol, m.p. 177 - 178.5°C.

(5) 1-(4-Acetamido-2-methoxyphenoxy)-3-[4-(3-chlorophenyl)piperazino]-2-propanol, m.p. 141 - 143.5°C.

(6) 1-(4-Acetamido-2-methoxyphenoxy)-3-[4-(2-chlorophenyl)piperazino]-2-propanol, m.p. 137.5 - 140°C.

(7) 1-(4-Acetamido-2-methoxyphenoxy)-3-[4-(4-fluorophenyl)piperazino]-2-propanol, m.p. 175.5 - 176.5°C.

(8) 1-(4-Acetamido-2-methoxyphenoxy)-3-[4-(3-fluorophenyl)piperazino]-2-propanol, m.p. 144.5 - 146°C, and its hydrochloride, m.p. 224 - 226°C.

(9) 1-(4-Acetamido-2-methoxyphenoxy)-3-[4-(3-trifluoromethylphenyl)piperazino]-2-propanol, m.p. 125 - 127°C.

Example 1

Polyisobutylene having a molecular weight of 40,000 (15 g) and polyisobutylene having a molecular weight of 2,000,000 (15 g) were dissolved in cyclohexane (1 liter), and thereto was added ethyl cellulose (ethoxy content: 48 %, viscosity: 90 cP) (20 g), and the mixture was dissolved by heating at 80°C. To the solution was dispersed a core material (100 g) as prepared in the same manner as described in the above Preparation (8), and the dispersion was cooled to room temperature with stirring at 250 rpm. The microcapsules thus obtain were separated by filtration, washed with n-hexane and dried. The microcapsules were passed through the JIS standard sieves (aperture: 500 and 105 $\mu$) to obtain microcapsule

preparation (108 g) containing 1-(4-acetamido-2-methoxyphenoxy)-3-[4-(3-fluorophenyl)piperazino]-2-propanol hydrochloride which conformed to the fine granule standard.

This preparation had a content of propanol derivative of 22.7 % and a time for releasing 50 % of the propanol derivative of 72 minutes (when it was kept in water at 37°C).

Example 2

In the same manner as described in Example 1 except that soy bean phosphatide (1 g) was used instead of polyisobutylene having a molecular weight of 40,000 (15 g) and polyisobutylene having a molecular weight of 2,000,000 (15 g), there was obtained a microcapsule preparation (113 g) containing 1-(4-acetamido-2-methoxyphenoxy)-3-[4-(3-fluorophenyl)piperazino]-2-propanol hydrochloride which conformed to the fine granule standard.

This preparation had a content of propanol derivative of 23.1 % and a time for releasing 50 % of the propanol derivative of 36 minutes (when it was kept in water at 37°C).

Example 3

Dimethylpolysiloxane (viscosity: 1,000 cSt at 25°C) (30 g) was dissolved in cyclohexane (1 liter), and thereto was added ethyl cellulose (ethoxy content: 47.2 %, viscosity: 110 cP) (10 g), and the mixture was

dissolved by heating at 80°C. To the solution was dispersed microparticles of 1-(4-acetamido-2-methoxyphenoxy)-3-[4-(3-fluorophenyl)piperazino]-2-propanol (250 g), which were passed through the JIS standard sieve (aperture: 149 μ ), and the dispersion was cooled to room temperature with stirring at 400 rpm. The microcapsule thus produced were separated by filtration, washed with n-hexane and dried. The microcapsules thus obtained were passed through the JIS stanard sieve (aperture: 350 μ ) to obtain a microcapsule preparation (250 g) containing 1-(4-acetamido-2-methoxyphenoxy)-3-[4-(3-fluorophenyl)piperazino)-2-propanol, which conformed to the powder standard as defined in the 10th Edition of the Pharmacopoeia of Japan.

This preparation had a content of propanol derivative of 96.2 % and a time of releasing 50 % of the propanol derivative of 56 minutes (when it was kept in water at 37°C) and of 5 minutes (when it was kept in a 1st fluid for disintegration test as defined in the Pharmacopoeia of Japan).

What is claimed is:

1. A microcapsule preparation for reducing increased intracranial pressure, which comprises a propanol derivative of the formula:

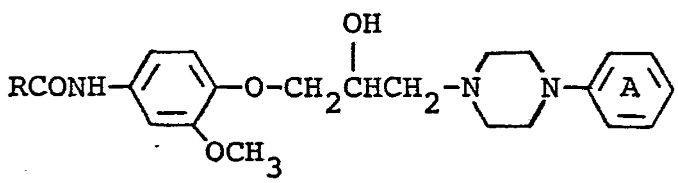
(I)

wherein R is an alkyl having 1 to 4 carbon atoms, and ring A is phenyl, halogenophenyl, methylphenyl or trifluoromethylphenyl, or a pharmaceutically acceptable acid addition salt thereof which is coated with ethyl cellulose film.

2. A microcapsule preparation according to claim 1, wherein the propanol derivative (I) or a pharmaceutically acceptable acid addition salt thereof is contained in an amount of 3 to 98 % by weight.

3. A microcapsule preparation according to claim 1, wherein the propanol derivative is a compound of the formula (I) wherein R is methyl, and the ring A is chlorophenyl or fluorophenyl, or a pharmaceutically acceptable acid addition salt thereof.

4. A microcapsule preparation according to claim 1, wherein the propanol derivative is a compound of the formula (I) wherein R is methyl, and the ring A is 2-chlorophenyl, 2-fluorophenyl, or 3-fluorophenyl, or a pharmaceutically acceptable acid addition salt thereof.

5. A microcapsule preparation according to claim 1, wherein the propanol derivative is 1-(4-acetamido-2-methoxyphenoxy)-3-[4-(3-fluorophenyl)-piperazino]-2-propanol, or a pharmaceutically acceptable acid addition salt thereof.

6. A process for preparing a microcapsule preparation for reducing increased intracranial pressure, which comprising dispersing a propanol derivative of the formula:

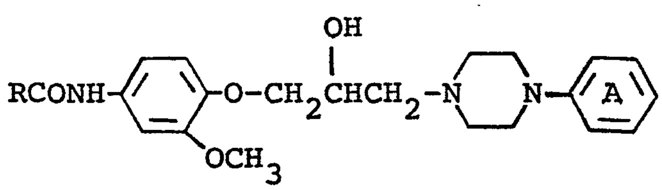

(I)

wherein R is alkyl having 1 to 4 carbon atoms, and ring A is phenyl, halogenophenyl, methylphenyl or trifluoromethylphenyl, or a pharmaceutically acceptable acid addition salt thereof into a hot solution of ethyl cellulose in an organic solvent, and cooling the dispersion, whereby forming a coating film of ethyl cellulose onto the propanol derivative (I) or a pharmaceutically acceptable acid addition salt thereof by phase separation phenomenon.

7. A process according to claim 6, wherein the organic solvent is a member selected from the group consisting of cyclohexane and a mixture of cyclohexane and n-hexane.

8. A process according to claim 7, wherein the organic solvent is cyclohexane.

9. A process according to claim 6, wherein the ethyl cellulose has an ethoxy content of 46.5 to 55 % and a viscosity of 3 to 500 cP.

10. A process according to claim 6 or 7, wherein the ethyl cellulose is used in an amount of 0.05 to 5 times by weight of the amount of the propanol derivative (I) or a pharmaceutically acceptable acid addition salt thereof.

11. A process according to any one of claim 6, 7, 8, 9, or 10, wherein the forming of ethyl cellulose coating film owing to phase separation phenomenon is carried out in the presence of a phase-separation-inducing agent.

12. A process according to claim 11, wherein the phase-separation-inducing agent is a member selected from the group consisting of polyethylene, butyl rubber, polybutadiene, polyisobutylene, and a mixture of two or more thereof.

13. A process according to claim 11 or 12, wherein the phase-separation-inducing agent is used in an amount of 0.1 to 10 % by weight based on the weight of the solution of ethyl cellulose.

14. A process according to any one of claim 6, 7, 8, 9, 10, 11, 12, or 13, wherein the forming of ethyl cellulose coating film is carried out in the presence of at least one additive selected from a wall film-forming auxiliary and a surface active agent.

15. A process according to claim 14, wherein the wall film-forming auxiliary is a member selected from the group consisting of at least one organic silicon polymer selected from dimethylpolysiloxane, methylphenylpolysiloxane, diphenylpolysiloxane, and polystyrene-polydimethylsiloxane block copolymer, and a mixture of at least one organic silicon polymer with at least one additive selected from silicon dioxide, titanium oxide and calcium stearate, and the surface active agent is a member selected from the group consisting of a sorbitan fatty acid ester, a glycerin fatty acid ester, a propylene glycol fatty acid ester, soy bean phosphatide, yolk phosphatide, calcium steryl-lactate, and a mixture of two or more thereof.

16. A process according to claim 14 or 15, wherein the wall film-forming auxiliary or surface active agent is used in an amount of 0.01 to 10 % by weight or about 0.003 to 10 % by weight, respectively, based on the weight of the solution of ethyl cellulose.

17. A process according to any one of claim 6, 7, 8, 9, 10, 11, 12, 13, 14, 15, or 16, wherein the propanol derivative (I) or a pharmaceutically acceptable acid addition salt thereof has a particle size of about 5 to 1,000 $\mu$.

0068324

Figure 1

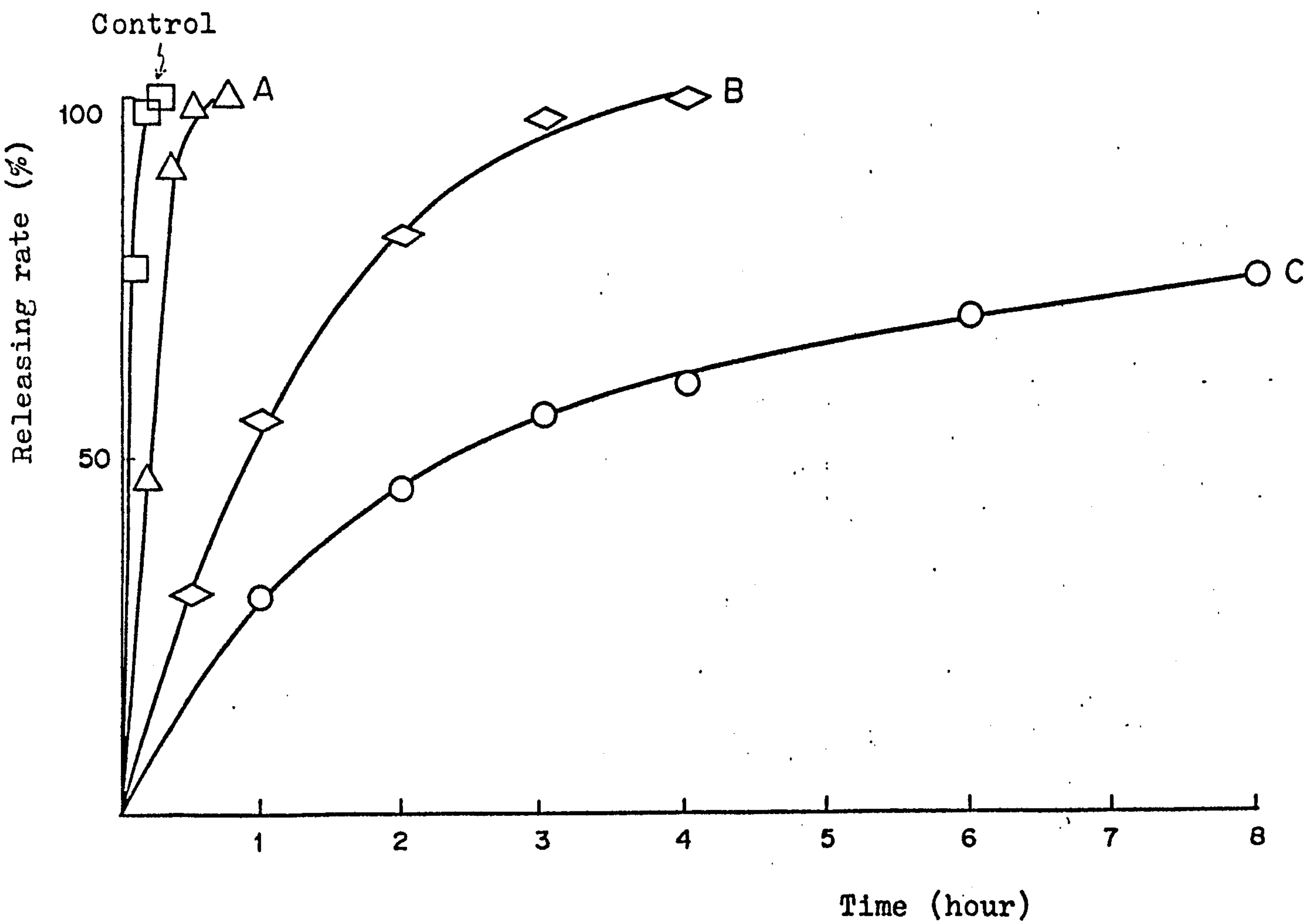


1/1